Europäisches Patentamt

(19) European Patent Office

Office européen des brevets

(11) Veröffentlichungsnummer: **0 131 821**
**A2**

(12) **EUROPÄISCHE PATENTANMELDUNG**

(21) Anmeldenummer: 84107563.3

(22) Anmeldetag: 29.06.84

(51) Int. Cl.⁴: **A 61 K 9/06**
**A 61 K 7/48**

(30) Priorität: 07.07.83 DE 3324415

(43) Veröffentlichungstag der Anmeldung:
23.01.85 Patentblatt 85/4

(84) Benannte Vertragsstaaten:
AT BE CH DE FR GB IT LI NL SE

(71) Anmelder: Beiersdorf Aktiengesellschaft
Unnastrasse 48
D-2000 Hamburg 20(DE)

(72) Erfinder: Sattler, Henning, Dr.
Jahnring 15
D-2000 Hamburg 60(DE)

(72) Erfinder: Asmussen, Bodo, Dr.
Dorotheenweg 1a
D-2071 Ammersbeck(DE)

(54) Fettsalbe.

(57) Gegenstand der Erfindung ist eine Fettsalbe, die
0 – 10% (a) Glycerin und/oder Polyäthylenglycol
0 – 10% (b) O/W-Emulgator oder Lösungsvermittler
vom nicht-ionischen Typ oder W/O-Emulgator
0,01 – 0,5% Puffersäure
0,05 – 1,0% Puffersalz
0 – 50% flüssiges Paraffin
50 – 95% Vaselin
etwa 0,5 – 1,5% Wasser und
0 – 10% Zusatzstoffe
enthält, wobei die Anteile von (a) und (b) nicht beide
zugleich 0% betragen.

Beiersdorf Aktiengesellschaft
Hamburg


Fettsalbe


Die vorliegende Erfindung bezieht sich auf eine neue Fettsalbe.


Zur Behandlung bestimmter Hautkrankheiten, die mit trockenen Hautzuständen einhergehen, dienen Fettsalben auf der Basis von Vaselin-, Paraffin- oder Polyäthylen-Kohlenwasserstoffen.


Ein Anwendungsgebiet ist dabei die Intervallbehandlung während der Corticosteroid-Therapie. Die hierfür bisher zur Verfügung stehenden Fettsalben-Präparate sind meist sogenannte Basis-Salben. Dies sind die wirkstofffreien Zubereitungen der Corticosteroid-Behandlung.


Es sind auch topische Zubereitungen in Form von Cremes, das heißt, von Emulsionen des O/W- und W/O-Typs mit einem Citronensäure/Citrat-Puffersystem bekannt. Sie ermöglichen die Wiederherstellung eines physiologischen Haut-pH-Wertes und enthalten den Citronensäure/Citratpuffer in der wäßrigen Phase gelöst. Diese Cremes sind jedoch aufgrund ihres hohen Wasseranteils bei trockener Haut nicht fettend genug.

-2-

Ferner sind noch lipophile Kohlenwasserstoff-Zubereitungen bekannt, in denen ebenfalls lipophile Wirkstoffe in gelöster Form enthalten sein können.

Aufgabe der Erfindung ist es, eine Fettsalbe auf der Grundlage von Vaselin-Paraffin-Kohlenwasserstoffen bereitzustellen, die ein Puffergemisch enthält. Die Fettsalbe soll das Puffergemisch in einer galenisch stabilen Form enthalten und geeignet sein, das Puffergemisch zur Entfaltung seiner - für die Anwendung - optimalen Wirksamkeit zu bringen.

Diese Aufgabe wird gelöst durch eine Fettsalbe, die dadurch gekennzeichnet ist, daß sie

| | |
|---|---|
| 0 - 10% (a) | Glycerin und/oder Polyäthylenglycol |
| 0 - 10% (b) | O/W-Emulgator oder Lösungsvermittler vom nicht-ionischen Typ oder W/O-Emulgator |
| 0,01 -0,5% | Puffersäure |
| 0,05 -1,0% | Puffersalz |
| 0 - 50% | flüssiges Paraffin |
| 50 - 95% | Vaselin |
| etwa 0,5 -1,5% | Wasser und |
| 0 - 10% | Zusatzstoffe |

enthält, wobei die Anteile von (a) und (b) nicht beide zugleich 0% betragen.

Vorzugsweise enthält die Fettsalbe

| | |
|---|---|
| 0,1 - 5% | Glycerin und/oder Polyäthylenglycol |
| 0,1 - 5% | O/W-Emulgator oder Lösungsvermittler vom nicht-ionischen Typ oder W/O-Emulgator |
| 0,1 -0,2% | Puffersäure |

| 0,1 - 0,7% | Puffersalz |
| 1 - 50% | flüssiges Paraffin |
| 50 - 95% | Vaselin |
| 0,8 - 1,2% | Wasser und |
| 0,1 - 3% | Zusatzstoffe, |

insbesondere aber

| 3% | Glycerin und/oder Polyäthylenglycol |
| 3% | O/W-Emulgator oder Lösungsver- |
| | mittler vom nicht-ionischen Typ |
| | oder W/O-Emulgator |
| 0,15% | Puffersäure |
| 0,5% | Puffersalz |
| 15,35% | flüssiges Paraffin |
| 75,5% | Vaselin |
| 1% | Wasser und |
| 1,5% | Zusatzstoffe. |

Mit der neuen erfindungsgemäßen Grundlage wird eine Fettsalbe vom Vaselin/Paraffin-Kohlenwasserstoff-Typ geschaffen, die das Puffergemisch in sehr fein verteilter Form enthält. Untersuchungen weisen darauf hin, daß sich - überraschenderweise - eine Lösung der ausgeprägt hydrophilen Pufferbestandteile in der rein lipophilen Salbengrundlage bildet. Dies hat den Vorteil, daß das Puffergemisch sofort - wie bei den wasserhaltigen Cremes - zur Verfügung steht. Außerdem kann es bei der Lagerung einer Lösung nicht zu dem von Suspensionszubereitungen bekannten Kristallwachstum kommen.

Als Glycerin kann praktisch wasserfreies Glycerin (ca. 1% Wassergehalt) oder auch wasserhaltiges Glycerin, vorzugsweise bis ca. 15% Wassergehalt gemäß den Angaben des Europäischen Arzneibuchs (Band 3), verwendet werden.

-4-

Das verwendete Polyäthylenglycol hat vorzugsweise ein Molekulargewicht von 200 bis 800, insbesondere etwa 400.

Glycerin und Polyäthylenglycol können allein oder im Gemisch miteinander verwendet werden.

Geeignete Emulgatoren und Lösungsmittler sind die bekannten O/W-Emulgatoren und Lösungsvermittler vom nicht-ionischen Typ ("nonionics") und W/O-Emulgatoren. Vorzugsweise werden Emulgatoren auf folgender Basis verwendet: a) ethoxylierte Sorbitanfettsäureester, b) Cetostearylalkoholpolyethylenglykolether, c) Polyoxyethylenfettsäureester, insbesondere Polyoxyethylenstearinsäureester, d) Polyethylenglykolstearyl/Cetylether, Glycerin-Polyethylenglykolricinoleat, Glycerin-Polyethylenglykoloxystearat, ethoxyliertes hydriertes Ricinusöl, Polyethylenglykolstearat, e) Polyoxyethylenglycerolfettsäureester, f) Polyäthylenglycole (Molekulargewicht bevorzugt 200 bis 800, insbesondere 400), g) Ethylhexansäurecetylstearylester, h) nichtionogene W/O-Emulgatoren, i) Partialglyceridgemische.

Sie sind unter den Handelsnamen a) "Tween", b) Cetomacrogel", c) "Myrj", d) "Cremophore", e) "Tagate" f) "Polidiol", g) "Luvitol", h) "Protegine", "Arlacele", i) "Softigen" bekannt.

Glycerinhaltige Zubereitungen, die frei von Polyäthylenglycol sind, enthalten vorzugsweise einen der vorstehend genannten Emulgatoren bzw. Lösungsvermittler, vorzugsweise in einer Menge von mindestens 1%.

Zubereitungen, die O/W-Emulgatoren oder Lösungsvermittler oder W/O-Emulgatoren (b) enthalten, vorzugsweise in einer Menge von mindestens 0,5%, insbesondere mindestens 1%, benötigen keinen Anteil (a) an Glycerin und Polyäthylenglycol. Zubereitungen, die (a) nicht enthalten, werden bevorzugt.

Von den Bestandteilsgruppen (a) Glycerin/Polyäthylenglycol und (b) O/W-Emulgator/Lösungsvermittler/ W/O-Emulgator, wobei a) und b) zusammen betrachtet werden, muß mindestens ein Vertreter in den erfindungsgemäßen Zubereitungen enthalten sein, vorzugsweise in einer Menge von mindestens 0,1%, insbesondere aber mindestens 1,0%. Die Prozentanteile der Gruppen (a) und (b) können also nicht gleichzeitig 0% betragen.

Wird Polyäthylenglycol allein oder im Gemisch mit Glycerin eingesetzt, ist es nicht erforderlich, O/W-Emulgatoren, Lösungsvermittler oder W/O-Emulgatoren zuzusetzen. Es ist aber vorzugsweise bei niederen Gewichtsanteilen dieser Substanzen zweckmäßig, die genannten Emulgatoren beziehungsweise Lösungsvermittler zu verwenden.

Dient Polyäthylenglykol als Emulgator beziehungsweise Lösungsvermittler, kann auf den Zusatz von Glycerin verzichtet werden. Kleine Glycerinanteile werden bevorzugt, insbesondere, wenn der Polyäthylenglycolanteil niedrig liegt. Besonders bevorzugt jedoch ist die alleinige Verwendung von Polyäthylenglycol, ohne Zusatz von Glycerin. Bevorzugte Emulgatoren/Lösungsvermittler (b), die ohne die Komponenten (a) verwendet werden, sind Polyäthylenglycol, nichtionogene W/O-Emulgatoren (insbesondere Protegin WX) und Partialglyceridgemische (insbesondere Softigen 767).

-6-

Geeignete Puffersäuren und -salze, die ein Puffergemisch bilden, sind (vorzugsweise schwache) Säuren und deren Salze, die eine deutliche Pufferwirkung sowohl für Säuren als auch für Alkalien besitzen. Bevorzugt werden physiologisch verträgliche Puffergemische. Besonders bevorzugt werden organische Säuren und deren Salze, insbesondere die an sich bekannten Citronensäure/Citrat- und Essigsäure/Acetat-Puffergemische.

Die neben der Citronensäure im Puffergemisch enthaltenen Citrate sind die Salze (und Hydrogensalze) der Citronensäure. Besonders geeignet sind die wasserlöslichen Citronensäuresalze, insbesondere die Alkalisalze und die Ammoniumsalze. Bevorzugt werden die Ammoniumsalze, insbesondere die Ammoniumhydrogencitrate. Besonders bevorzugt wird das Diammoniumhydrogencitrat. Ebenfalls werden die Alkali- und Ammoniumacetate bevorzugt.

Der Vaselin-Paraffin-Kohlenwasserstoffanteil kann ausschließlich aus Vaselin bestehen oder zur Erzielung einer weicheren Konsistenz aus einem Gemisch von Vaselin und flüssigem Paraffin. Vorzugsweise beträgt der Anteil an flüssigem Paraffin 0,5 bis 50%, insbesondere 5 bis 25% der Fettsalbe. Es können auch höhermolekulare Anteile, wie z.B. Polyäthylen, enthalten sein.

Es ist erforderlich, daß die erfindungsgemäße Fettsalbe eine geringe Menge Wasser enthält. Dieser Wasseranteil beträgt etwa 0,5% bis 1,5%. Die angegebenen Grenzen können etwas über- oder unterschritten werden. Beispielsweise führen Schwankungen des Wasseranteils des Glycerins zu kleineren oder größeren Anteilen. Grundsätzlich jedoch ist der Wasseranteil des verwendeten Glycerins bei der Berechnung des Wasseranteils zu berücksichtigen. Vorzugsweise beträgt der Wasseranteil etwa 0,8 bis 1,2%, insbesondere etwa 1%.

Ferner kann die erfindungsgemäße Fettsalbe auch mit Vaselin-Paraffin-Kohlenwasserstoffen verträgliche Zusätze enthalten. Vorzugsweise beträgt der Anteil der Zusätze etwa 0,1 bis 10%, insbesonders bevorzugt 1 bis 5%, insbesondere 1,5%. Geeignet sind Substanzen wie Cetylstearylalkohol, Walrat und Bienenwachs. Auch Konservierungsmittel können in den üblichen Mengen verwendet werden.

Zur Herstellung werden Vaselin-Paraffin-Kohlenwasserstoff-Bestandteile, gegebenenfalls Emulgator/-Lösungsvermittler, Glycerin, Polyäthylenglycol und Wasser sowie eventuelle Zusatzstoffe auf 75° - 95° erhitzt, dann Puffersäure und Puffersalz (beide pulverförmig) zugegeben und gerührt, bis eine klare Lösung entsteht. Das Rühren wird beim Abkühlen bis zum Erstarren der Masse fortgesetzt.

Ein weiteres Herstellungsverfahren besteht darin, daß man zu den auf 75° - 95° erhitzten Vaselin-Paraffin-Kohlenwasserstoff-Bestandteilen (A) eine Lösung oder Mischung (B) von Wasser und Puffergemisch gibt, die gegebenenfalls Glycerin, Polyäthylenglycol und Emulgator/Lösungsvermittler zugemischt enthält. Auch die (A) zuzufügenden übrigen Bestandteile (B) können auf 75° - 95° erhitzt und dann zugegeben werden. Anschließend wird gerührt, bis eine klare Lösung entsteht. Das Rühren wird beim Abkühlen bis zum Erstarren der Masse fortgesetzt. Dieses zweite Verfahren wird vorteilhaft dann verwendet, wenn Wasser extra zugegeben

-8-

wird.Zusatzstoffe können in (A) und (B) enthalten sein. Glycerin enthaltende Zubereitungen werden auch vorteilhaft erhalten, indem die gegebenenfalls zuzufügende Wassermenge zunächst mit Glycerin vermischt wird und diese Lösung dann wie im vorstehend beschriebenen ersten Verfahren mit den Vaselin-Paraffin-Kohlenwasserstoffen erhitzt wird.

Alle Mengenangaben, Anteile und Prozentanteile sind auf das Gewicht bezogen.

Die erfindungsgemäße Fettsalbe mit dem Puffergemisch wird besonders vorteilhaft in der Intervallbehandlung während der Corticosteroid-Therapie angewendet. Sie wird aber bevorzugt auch zur Hautbehandlung, insbesondere zur Regulierung des natürlichen pH-Wertes der Haut verwendet.

Die folgenden Beispiele dienen zur Erläuterung der Erfindung:


Beispiel 1


Mit den angegebenen Bestandteilen wird eine Fettsalbe hergestellt:

| | |
|---|---|
| Weißes Vaselin | 90,84% |
| Glycerin (Wassergehalt 15%) | 4,00% |
| Emulgator (Polyoxyethylen-(20)sorbitanmonostearat, "Tween 60") | 5,00% |
| Citronensäure, pulv. | 0,01% |
| Diammoniumhydrogencitrat, pulv. | 0,15% |

-9-

Das Vaselin, Glycerin und der Emulgator werden auf 75°C erhitzt. Dann werden die Citronensäure und das Diammoniumhydrogencitrat unter Rühren zugegeben und gelöst. Anschließend wird bis zum Erstarren der Masse weitergerührt.

Beispiel 2

Mit den angegebenen Bestandteilen wird eine Fettsalbe hergestellt:

| | |
|---|---|
| Weißes Vaselin | 72,85% |
| dickflüssiges Paraffin | 15,00% |
| Cetylstearylalkohol | 1,50% |
| Glycerin, wasserfrei | 5,00% |
| Emulgator (Polyoxyethylen 40 stearat, "Myrj 52") | 5,00% |
| Citronensäure, pulv. | 0,15% |
| Diammoniumhydrogencitrat, pulv. | 0,50% |
| Wasser, gereinigt | 0,50% |

Vaselin, dickflüssiges Paraffin, Cetylstearylalkohol, Glycerin (wasserfrei) und der Emulgator werden auf 80°C erhitzt. Dann werden Citronensäure und das Diammoniumhydrogencitrat, beide vermischt mit dem Wasser, zugegeben. Es wird bis zum Lösen gerührt und das Rühren bis zum Festwerden der Masse (salbenartige Konsistenz) fortgesetzt.

-10-

Beispiel 3

Mit den angegebenen Bestandteilen wird eine Fettsalbe hergestellt:

| | |
|---|---|
| Weißes Vaselin | 72,85% |
| dickflüssiges Paraffin | 15,00% |
| Cetylstearylalkohol | 1,50% |
| Glycerin (Wassergehalt 15%) | 5,00% |
| Emulgator (Polyoxyethylen-40-stearat, "Myrj 52") | 5,00% |
| Essigsäure, 100% | 0,15% |
| Natriumacetat·3H$_2$O, pulv. | 0,50% |

Vaselin, dickflüssiges Paraffin, Cetylstearylalkohol, Glycerin und der Emulgator werden auf 80°C
erhitzt. Dann werden Essigsäure und Natriumacetat
bei 60°C zugegeben. Es wird bis zum Lösen gerührt
und das Rühren bis zum Festwerden der Masse (salbenartige Konsistenz) fortgesetzt.

Beispiel 4

Mit den angegebenen Bestandteilen wird eine Fettsalbe hergestellt:

| | |
|---|---|
| Weißes Vaselin | 82,35% |
| dickflüssiges Paraffin | 15,00% |
| Polyäthylenglycol 400 | 1,00% |
| gereinigtes Wasser | 1,00% |
| Citronensäure, pulv. | 0,15% |
| Diammoniumhydrogencitrat, pulv. | 0,50% |

-11-

Das Vaselin und das dickflüssige Paraffin werden auf 75°C erhitzt. Citronensäure und Diammoniumhydrogencitrat werden in Wasser gelöst und mit Polyäthylenglycol 400 vermischt. Die beiden Phasen werden vereinigt und kaltgerührt.


Beispiel 5


Mit den angegebenen Bestandteilen wird eine Fettsalbe hergestellt:

| | |
|---|---|
| Weißes Vaselin | 80,85% |
| dickflüssiges Paraffin | 15,00% |
| Polyäthylenglycol 400 | 0,50% |
| 2-Ethylhexansäurecetylstearyl- | |
| ester (Luvitol ® EHO) | 2,00% |
| gereinigtes Wasser | 1,00% |
| Citronensäure, pulv. | 0,15% |
| Diammoniumhydrogencitrat, pulv. | 0,50% |

Das Vaselin und das dickflüssige Paraffin werden auf 75°C erhitzt. Citronensäure und Diammoniumhydrogencitrat werden in Wasser gelöst und mit Polyäthylenglycol und 2-Ethylhexansäurecetylstearylester vermischt. Die beiden Phasen werden vereinigt und kaltgerührt.


Beispiel 6


Mit den angegebenen Bestandteilen wird eine Fettsalbe hergestellt:

| | |
|---|---|
| Weißes Vaselin | 78,35% |
| dickflüssiges Paraffin | 15,00% |

-12-

nichtionogener W/O-Emulgator
(Protegin® WX)                5,00%
gereinigtes Wasser              1,00%
Citronensäure, pulv.          0,15%
Diammoniumhydrogencitrat, pulv.    0,50%

Das Vaselin und das dickflüssige Paraffin werden auf 75°C erhitzt. Citronensäure und Diammoniumhydrogencitrat werden in Wasser gelöst, dann wird der W/O-Emulgator zugesetzt und alles auf 75°C erhitzt. Die beiden Phasen werden vereinigt und kaltgerührt.

Beispiel 7

Mit den angegebenen Bestandteilen wird eine Fettsalbe hergestellt:

Weißes Vaselin              81,35%
dickflüssiges Paraffin        15,00%
Partialglyceridgemisch
(Softigen® 767)             2,00%
gereinigtes Wasser             1,00%
Citronensäure, pulv.         0,15%
Diammoniumhydrogencitrat      0,50%

Das Vaselin und das dickflüssige Paraffin werden auf 75°C erhitzt. Citronensäure und Diammoniumhydrogencitrat werden in Wasser gelöst und mit dem Partialglyceridgemisch vermischt. Die beiden Phasen werden vereinigt und kaltgerührt.

– 13 –

Patentansprüche

1. Fettsalbe, dadurch gekennzeichnet, daß sie

| | | |
|---|---|---|
| 0 – 10% | (a) | Glycerin und/oder Polyäthylen-glycol |
| 0 – 10% | (b) | O/W-Emulgator oder Lösungsver-mittler vom nicht-ionischen Typ oder W/O-Emulgator |
| 0,01 – 0,5% | | Puffersäure |
| 0,05 – 1,0% | | Puffersalz |
| 0 – 50% | | flüssiges Paraffin |
| 50 – 95% | | Vaselin |
| etwa 0,5 – 1,5% | | Wasser und |
| 0 – 10% | | Zusatzstoffe |

enthält, wobei die Anteile von (a) und (b) nicht beide zugleich 0% betragen.

2. Fettsalbe gemäß Anspruch 1, dadurch gekennzeichnet, daß sie

| | |
|---|---|
| 0,1 – 5% | Glycerin und/oder Polyäthylen-glycol |
| 0,1 – 5% | O/W-Emulgator oder Lösungsver-mittler vom nicht-ionischen Typ oder W/O-Emulgator |
| 0,1 – 0,2% | Puffersäure |
| 0,1 – 0,7% | Puffersalz |
| 1 – 50% | flüssiges Paraffin |
| 50 – 95% | Vaselin |
| 0,8 – 1,2% | Wasser und |
| 0,1 – 3% | Zusatzstoffe |

enthält.

3. Fettsalbe gemäß Anspruch 1 oder 2, dadurch gekennzeichnet, daß die Puffergemische Citronensäure/Citrat und Essigsäure/Acetat sind.